Europäisches Patentamt

European Patent Office

Office européen des brevets

(19)

(11) Veröffentlichungsnummer: **0 223 221 B1**

# EUROPÄISCHE PATENTSCHRIFT

(12)

(45) Veröffentlichungstag der Patentschrift: **15.04.92**

(21) Anmeldenummer: **86115902.8**

(22) Anmeldetag: **15.11.86**

(51) Int. Cl.5: **C12N 11/10**, C12N 11/08, C12Q 1/28, C12N 9/96

(54) **Wasserlösliche, stabilisierte Peroxidasederivate, Verfahren zu ihrer Herstellung und Verwendung zur Bestimmung von Wasserstoffperoxid.**

(30) Priorität: **21.11.85 DE 3541186**

(43) Veröffentlichungstag der Anmeldung:
**27.05.87 Patentblatt 87/22**

(45) Bekanntmachung des Hinweises auf die Patenterteilung:
**15.04.92 Patentblatt 92/16**

(84) Benannte Vertragsstaaten:
**AT BE CH DE ES FR GB GR IT LI LU NL SE**

(56) Entgegenhaltungen:
**EP-A- 0 049 475**
**EP-A- 0 069 379**

**CHEMICAL ABSTRACTS, Band 100, Nr. 13, 26. März 1984, Zusammenfassung Nr. 101237w, Seite 474, Columbus, Ohio, US; P. TYSSEN et al.: "Highly efficient and simple methods for the preparation of peroxidase and active peroxidase-antibody conjugates for enzyme immunoassays", & ANAL. BIOCHEM. 1984, 136(2), 451-7**

(73) Patentinhaber: **BOEHRINGER MANNHEIM GMBH**
**Sandhofer Strasse 116**
**W-6800 Mannheim 31(DE)**

(72) Erfinder: **Tischer, Wilhelm, Dr. Dipl.-Ing.**
**Finkenweg 5**
**W-8123 Peissenberg(DE)**
Erfinder: **Heinle, Josef**
**Bayrischzeller Strasse 29**
**W-8000 München 90(DE)**
Erfinder: **Town, Michael-Harold, Dr.rer.nat.**
**Waldstrasse 45**
**W-8125 Oberhausen(DE)**

THE WORLD BIOTECH REPORT '84, Band 1: Europe, The Proceedings of Biotech '84 Europe Conference, Seiten 379-390, Online Publicatons Ltd, Pinner Green House, Ash Hill Dr., Pinner, Middx, GB; P. MONSAN: "Stabilization of enzyme activity"

DIE ANGEWANDTE MAKROMOLEKUALRE CHEMIE, Band 104, Nr. 1638, April 1982, Seiten 129-143, Hüthig & Wepf Verlag, Heidelberg, DE; L. D'ANGIURO et al.: "Bisacryloylpiperazine as vinylating agent for enzyme immobilization by graft-copolymerization onto polysaccharides

DIE MAKROMOLEKULARE CHEMIE, Band 182, Nr. 6, Juni 1981, Seiten 1641-1648, Heidelberg, DE; R.H. REINER et al.: "HIO4-oxidized soluble polysaccharides as polyfunctional links for covalent binding of enzymes, 1"

ANALYTICAL BIOCHEMISTRY, Band 136, Nr. 2, 1. Februar 1984, Seiten 451-457, Academic Press, Inc.: P. TIJSSEN et al.:"Highly efficient and simple methods for the preparation of peroxidase and active peroxidase-antibody conjugates for enzyme immunoassays"

EP 0 223 221 B1

**Beschreibung**

Die Erfindung betrifft eine wasserlösliche, stabilisierte Peroxidase (POD), ein Verfahren zu ihrer Herstellung und ihre Verwendung zur Bestimmung von Wasserstoffperoxid.

Peroxidase (E.C. 1.11.1.7) ist ein in diagnostischen Reagentien häufig eingesetztes Enzym. Es wird insbesondere verwendet zur Bestimmung von Wasserstoffperoxid, welches in Oxidasen-katalysierten Reaktionen entstanden ist. Hierbei erfolgt eine oxidative Kupplung von Farbkomponenten zu einem farbigen Produkt, dessen Menge einen quantitativen Rückschluß auf das zu bestimmende Enzym oder Substrat gibt.

POD ist in Pufferlösungen weitgehend stabil, d.h. die Aktivität nimmt auch bei mehrtägiger Lagerung nicht oder nur geringfügig ab. In den üblichen Reagentienzusammensetzungen für die klinische Chemie, welche Detergentien und/oder Komplexbildner, wie z. B. EDTA,enthalten, ist die POD jedoch instabil. Üblicherweise rechnet man mit Verlusten von > 9O % der Aktivität in 6 Tagen bei Raumtemperatur. Um sicherzustellen, daß die POD-Aktivität auch nach mehrtägiger Lagerung solcher Reagentien noch in ausreichendem Maß vorhanden ist, wird üblicherweise Peroxidase in einem mehrfachen Überschuß eingesetzt.

Ein wesentlicher Nachteil von solchen hohen POD-Konzentrationen ist jedoch, daß dadurch beträchtliche Reagentienleerwerte entstehen. Verursacht wird dies durch Haemgruppen, welche das Enzym enthält und die im sichtbaren Bereich Licht absorbieren. Dies führt zu Ungenauigkeiten der Meßergebnisse.

Aus DE 29 19 622 ist ein Verfahren zur Herstellung von in detergenshaltigen Lösungen stabilen, wasserlöslichen Enzymderivaten bekannt. Hierbei werden die Enzyme mit aldehydgruppenhaltigen Polysaccharid-Derivaten umgesetzt. Im Gegensatz zu anderen auf diese Weise modifizierten Enzymen, die so stabilisiert werden können, zeigt derart modifizierte Peroxidase jedoch keine ausreichend verbesserte Stabilität in Detergens- und/oder Komplexbildner-haltigen Lösungen.

Die Aufgabe der vorliegenden Erfindung besteht demzufolge darin, wasserlösliche Peroxidase-Derivate bereitzustellen, deren Aktivität in wäßrigen Detergens- und/oder Komplexbildner-haltigen Lösungen über längere Zeit erhalten bleibt.

Gelöst wird diese Aufgabe erfindungsgemäß durch ein wasserlösliches Peroxidase-Derivat, welches erhältlich ist durch Oxidation der Peroxidase mit Perjodsäure oder einem ihrer Salze und Bindung an ein wasserlösliches Polymeres aus der Gruppe Polysaccharid, Polyethylenglykol, Polyvinylpyrrolidon und Polysäureanhydrid.

Überraschenderweise hat sich nämlich gezeigt, daß derart modifizierte POD-Derivate eine ausgezeichnete Langzeitstabilität in Detergens- und/oder Komplexbildner-haltigen Lösungen zeigen, ohne daß wesentliche Enzymeigenschaften verloren gehen.

Die Oxidation der Peroxidase erfolgt unter den dem Fachmann geläufigen Bedingungen mit Perjodsäure oder einem ihrer Salze, insbesondere mit Alkalisalzen. Dies ist beispielsweise im Rahmen der Herstellung von Peroxidase-Antikörper-Konjugaten beschrieben (E. ISHIKAWA, J. Immunoassay 4, 2O9 - 327 (1983)). Perjodsäure oder Perjodat werden hierbei bevorzugt im Überschuß angewendet. Besonders bevorzugt ist ein 2O - 2OOfacher Überschuß. Die Temperatur und der pH-Wert können im weiten Bereich variieren. Es hat sich jedoch gezeigt, daß im schwach Sauren (pH 5-6) und unter Kühlung (O° 1O°C) die Reaktion mit besonders guten Ausbeuten abläuft. Nach Durchführung der Oxidation ist es vorteilhaft, das Oxidationsmittel durch eine Dialyse oder einen Chromatographieschritt (z. B. an Sephadex® G-25) zu entfernen.

In einem weiteren Schritt wird das oxidierte Enzym an ein wasserlösliches Polymeres gebunden. Die Kupplung mit diesem Polymeren erfolgt in an sich bekannter Weise, indem man die oxidierte POD in wäßriger Lösung mit dem gelösten Polymeren, unter definierten Bedingungen, umsetzt (vgl. z. B. EP O O69 379).

Als Polymere geeignet sind wasserlösliche Polysaccharide, Polyäthylenglykole, Polyvinylpyrrolidone oder Polysäureanhydride. Ein bevorzugtes Polysaccharid ist Dextran. Sehr gute Ergebnisse werden jedoch auch mit anderen wasserlöslichen Polysacchariden, wie insbesondere mit löslicher Stärke,erzielt. Auch hochmolekulare Polymere von Mono- und Disacchariden, wie sie beispielsweise durch Umsetzung mit Epihalogenhydrin (z. B. Ficoll®) erhalten werden, erwiesen sich als gut geeignet. Als Polysäureanhydrid erwies sich als besonders geeignet ein Copolymer aus Methylvinyläther mit Maleinsäureanhydrid.

Die Bindung der oxidierten POD an das Polymere kann ohne weitere Zwischenschritte erfolgen, sofern das Polymere reaktive Gruppen besitzt (z. B. Aminogruppen), die mit der oxidierten POD reagieren. Sofern das Polymere keine reaktiven Gruppen trägt, wird es vor Umsatz mit der oxidierten POD vorteilhaft aktiviert und mit reaktiven Gruppen versehen. Derartige Methoden sind dem Fachmann geläufig und beispielsweise in EP O O69 379 bzw. bei J.K. Inman, J. Immunol. 114, 7O4 (1975) beschrieben.

Zur Aktivierung von Polysacchariden erwies sich die Umsetzung mit Bromcyan, 1-Cyano-4-dimethylaminopyridiniumsalz (CDAP) oder 2,4,6-Trichlor-1,3,5-Triazin (TCT) als vorteilhaft. Zur Aktivierung von Polyäthylenglykol ist die Umsetzung mit TCT besonders geeignet. Ebenso können mit Carboxylgruppen beladene Polymere mit N-Hydroxy-Succinimid zur Aktivierung umgesetzt werden.

In einer bevorzugten Ausführungsform wird die oxidierte POD über einen oder mehrere Alkylenreste mit 1 - 8 C-Atomen im Molekül an das Polymere gebunden. Hierzu wird die oxidierte POD mit dem entsprechenden Alkylendiamin umgesetzt und dann an das gegebenenfalls aktivierte Polymere gebunden. Besonders bevorzugt als Alkylendiamin ist Ethylendiamin.

Es ist vorteilhaft, zur Stabilisierung der Bindung mit dem Alkylendiamin, vor Umsetzung mit dem Polymeren einen Reduktionsschritt, bevorzugt mit Borhydrid oder Cyan-Borhydrid, anzuschließen und ggf. das modifizierte Enzym über einen Dialyse- oder Chromatographieschritt aufzureinigen.

Die Kupplung erfolgt zweckmäßig bei Temperaturen zwischen 0° und Raumtemperatur und pH-Werten zwischen 6 und 10. Das Molverhältnis Enzym zu Polymerem kann in weiten Bereichen variiert werden (1 : 1 - 1 : 20). Bei einem Molverhältnis von ca. 1 : 4 erhält man jedoch eine besonders hohe Ausbeute an Kupplungsprodukt.

Die Isolierung der erfindungsgemäßen POD-Derivate kann nach Abschluß der Kupplungsreaktion, beispielsweise durch Zusatz von Aceton und Ausfällung, erfolgen. Bevorzugt wird jedoch die erhaltene Lösung lyophilisiert, zweckmäßig nach vorheriger Reinigung durch Dialyse oder Chromatographie.

Die Derivatisierung der POD hat keinen nachteiligen Einfluß auf die Spezifität und Aktivität des Enzyms. Im Gegenteil wurde überraschenderweise beobachtet, daß die Michaeliskonstante (Substrat Wasserstoffperoxid) der erfindungsgemäßen POD-Derivate deutlich kleiner ist, als die der nicht derivatisierten POD. Die Derivate können vorteilhaft zur Bestimmung von Wasserstoffperoxid, beispielsweise in Reagentien für klinisch-chemische Bestimmungen (z. B. Harnsäure) verwendet werden. Durch die kleinere Michaeliskonstante und die verbesserte Stabilität in Detergens-und/oder Komplexbildner-haltigen Reagenzlösungen kann der Gehalt an erfindungsgemäßen POD-Derivaten wesentlich geringer sein als bei Verwendung von nicht derivatisierter POD.

Die nachfolgenden Beispiele erläutern die Erfindung weiter.

Beispiel 1: Bindung von aktivierter Peroxidase an Dextran

a) Aktivierung der POD

5 g Meerrettich-Peroxidase (E.C. 1.11.1.7) werden in 250 ml Acetatpuffer (30 mmol/l, pH 5,5) gelöst, 37,5 ml NaJO$_4$ (0,2 mol/l) zugegeben und 40 min. bei Raumtemperatur inkubiert. Anschließend werden 37,5 ml 1 mol/l Ethylenglykol zugegeben und weitere 20 min. bei Raumtemperatur inkubiert. Danach wird die Reaktionsmischung über eine Sephadex G-25-Säule gereinigt (Laufmittel: Natriumacetatpuffer pH 5,5, 10 mmol/l).

b) Umsetzung mit Ethylendiamin

Mit 1 mol/l Natrium-Carbonat/Natrium-Bicarbonatpuffer, pH 9,8, wird der pH-Wert des Eluats auf 9,5 eingestellt. Anschließend werden 11,25 ml 1 mol/l Ethylendiamin-Lösung, pH 9,5, zugegeben. Anschließend wird eine Stunde bei 22°C inkubiert, wobei mit dem o. g. Carbonat/Bicarbonatpuffer der pH bei 9,5 gehalten wird (pH-Stat).

Der Ansatz wird mit 1 mol/l TRA-Puffer[1], pH 7,0, auf pH 8,0 eingestellt. Anschließend werden 750 mg Natrium-Cyano-Borhydrid zugesetzt und 15 Minuten bei 22°C inkubiert, wobei der pH-Wert mit TRA-Puffer auf pH 8,0 gehalten wird (pH-Stat). Danach wird die Reaktionsmischung über eine Sephadex-G-25-Säule gereinigt (Laufmittel: 10 mmol/l Phosphatpuffer pH 7,0).

c) Bindung an Dextran

Die nach b) erhaltene oxidierte POD wird an CDAP-BF$_4$-aktiviertes Dextran T 40 nach dem in J. J. Marshall, Preservation of Enzymes by Conjugation with Dextran
American Chemical Society Symposium Series 123 (1980) 125 - 140 beschriebenen und nach J. Kohn und M. Wilchek, The Use of Cyanogen Bromide and other novel Cyanylating Agents for the Activation of Polysaccharide Resins, Appl. Biochem. Biotech. 9 (1984) 285 - 305 modifizierten Verfahren gebunden. Man erhält eine spezifische POD-Aktivität von 15 - 18 U/mg.

[1] TRA: Triäthanolamin

4

Beispiel 2: Bindung von Peroxidase an Ficoll®

Wie in Beispiel 1 beschrieben wird Peroxidase derivatisiert und mit Ficoll® anstelle von Dextran T 4O umgesetzt. Man erhält als Ausbeute: 25 - 3O g Lyophilisat mit einer spezifischen POD-Aktivität von 14 - 17 U/mg.

Beispiel 3: Bindung an Aminodextran

Die nach Beispiel 1b) hergestellte Reaktionsmischung wird mit 25 g Aminodextran (hergestellt nach J.K. Inman, J. Immunol. 114, 7O4 (1975)) (Molekulargewicht ~ 4OOOO) versetzt und 2 Stunden inkubiert bei Raumtemperatur und pH 9,3. Anschließend wird, wie in Beispiel 1b, bei pH 8,O mit 75O mg Natrium-Borhydrid reduziert und,wie in Beispiel 1 beschrieben, gereinigt. Als Ausbeute erhält man 25 - 3O g Lyophilisat mit einer POD-Aktivität von 9 - 14 U/mg.

Beispiel 4: Bindung von nativer POD an Dextran

Meerrettich-Peroxidase wird, wie in Beispiel 1c beschrieben, direkt an $CDAP-BF_4$-aktiviertes Dextran T 4O gebunden.

Beispiel 5: Vergleich der Stabilität von verschiedenen POD-Derivaten

| Enzym | % Restaktivität in Reagenz a nach 6 Tagen bei 20°C |
|---|---|
| POD nativ | 4 % |
| nach Beispiel 4 | 11 % |
| nach Beispiel 3 | 64 % |
| nach Beispiel 2 | 70 % |
| nach Beispiel 1 | 70 % |

Zusammensetzung Reagenz a:  0,1 mol/l k-phosphat-Puffer (pH 8,2)
0,1 % $NaN_3$
0,1 % EDTA
0,5 % Lutensol ON 5O
7 mmol/1 Na-Cholat

Es zeigt sich, daß die Stabilität von nicht derivatsierter (nativer) POD sowie von ohne Derivatisierung Dextranfixierter POD (Beispiel 4) nur gering ist, während die Stabilität von POD, welche nach Oxidation Dextran-fixiert wurde, deutlich verbessert ist.

Beispiel 6

Die Stabilität von POD wurde in Abhängigkeit von Detergens und EDTA-Zusatz untersucht. Hierbei ergab sich (Inkubationsdauer 16 Stunden bei 37°C):

| Reagenzzusammensetzung | Restaktivität | |
|---|---|---|
| | POD nativ | POD nach Bsp. 1 |
| 1. 0,1 mol/l TRA-puffer pH 8,2 | 78 % | 80 % |
| 2. ① + 0,1 % EDTA[1]) | 5 % | 18 % |
| 3. 0,1 mol/l K-Phosphat-Puffer | 75 % | 85 % |
| 4. ③ + 0,5 % Lutensol ON 50 + 7 mmol/1 NaCholat [1]) | 3 % | 27 % |
| 5. ③ + 0,1 % EDTA | 23 % | 57 % |
| 6. ④ + 0,1 % EDTA [1]) | 8 % | 43 % |

[1]) Untersuchungen werden in Reagenzmix zusammen mit anderen Bestandteilen (NaN$_3$ + Farbstoffe) durchgeführt.

Beispiel 7

Als Michaeliskonstante erhält man (Substrat Wasserstoffperoxid) nach der Lineweaver-Burk-Methode im Reagenzmix nach Beispiel 5:

| Enzym | $K_M$ |
|---|---|
| POD nativ | $1,76 . 10^{-5}$ |
| nach Bsp. 1 | $7,12 . 10^{-6}$ |

**Patentansprüche**

1. Wasserlösliches Peroxidase-Derivat, erhältlich durch Oxidation der Peroxidase mit Perjodsäure oder einem ihrer Salze und Bindung über einen oder mehrere Alkylenreste mit 1 - 8 C-Atomen an ein wasserlösliches Polymeres aus der Gruppe Polysaccharid, Polyäthylenglykol, Polyvinylpyrrolidon und Polysäureanhydrid.

2. Wasserlösliches Peroxidase-Derivat nach Anspruch 1, dadurch gekennzeichnet, daß der Alkylenrest ein Ethylenrest ist.

3. Wasserlösliches Peroxidase-Derivat nach Anspruch 1 oder 2, dadurch gekennzeichnet, daß das Polymere ein Polysaccharid ist.

4. Verfahren zur Herstellung eines wasserlöslichen Peroxidase-Derivats, dadurch gekennzeichnet, daß man Peroxidase mit Perjodsäure oder einem ihrer Salze oxidiert, mit einem Alkylendiamin mit 1 - 8 C-Atomen umsetzt und an ein wasserlösliches Polymeres aus der Gruppe Polysaccharid, Polyäthlyenglykol, Polyvinylpyrrolidon oder Polysäureanhydrid bindet.

5. Verfahren nach Anspruch 4, dadurch gekennzeichnet, daß die Bindung an ein aktiviertes Polymeres erfolgt.

6. Verfahren nach Anspruch 4 oder 5, dadurch gekennzeichnet, daß als Polymeres Polysaccharid verwendet wird.

7. Verfahren nach Anspruch 4, dadurch gekennzeichnet, daß man als Alkylendiamin Ethylendiamin und als Polymeres aktiviertes Polysaccharid verwendet.

8. Verwendung eines wasserlöslichen Peroxidase-Derivats nach einem der Ansprüche 1 bis 3 zur H$_2$O$_2$-Bestimmung.

**Claims**

6

1. Water-soluble peroxidase derivative obtainable by the oxidation of peroxidase with periodic acid or one of its salts and binding to a water-soluble polymer from the group polysaccharide, polyethylene glycol, polyvinylpyrrolidone and polyacid anhydride via one or more alkylene radicals with 1 - 8 C-atoms.

2. Water-soluble peroxidase derivative according to claim 2, characterised in that the alkylene radical is an ethylene radical.

3. Water-soluble peroxidase derivative according to claim 1 or 2, characterised in that the polymer is a polysaccharide.

4. Process for the preparation of a water-soluble peroxidase derivative, characterised in that one oxidises peroxidase with periodic acid or one of its salts, reacts with an alkylenediamine with 1 - 8 C-atoms and binds to a water-soluble polymer from the group polysaccharide, polyethylene glycol, polyvinylpyrrolidone or polyacid anhydride.

5. Process according to claim 4, characterised in that the binding takes place to an activated polymer.

6. Process according to claim 4 or 5, characterised in that polysaccharide is used as polymer.

7. Process according to claim 4, characterised in that one uses ethylenediamine as alkylenediamine and activated polysaccharide as polymer.

8. Use of a water-soluble peroxidase derivative according to one of claims 1 to 3 for the determination of $H_2O_2$.

## Revendications

1. Dérivé de peroxydase soluble dans l'eau qui peut être obtenu par oxydation de la peroxydase avec un acide périodique ou l'un de ses sels et par liaison par l'intermédiaire d'un ou plusieurs restes d'alcoylène comportant 1 à 8 atomes-C sur un polymère soluble dans l'eau choisi dans le groupe des polysaccharide, polyéthylèneglycol, polyvinylpyrrolidone et polyacide anydrique.

2. Dérivé de peroxydase soluble dans l'eau selon la revendication 1, caractérisé en ce que le reste d'alcoylène est un reste d'éthylène.

3. Dérivé de peroxydase soluble dans l'eau selon la revendication 1 ou 2, caractérisé en ce que le polymère est un polysaccharide.

4. Procédé pour la préparation d'un dérivé de peroxydase soluble dans l'eau caractérisé en ce que l'on oxyde la peroxydase avec un acide périodique ou l'un de ses sels, en ce que l'on fait réagir la peroxydase avec un alkylènediamine comportant 1 à 8 atomes-C et en ce que l'on fixe celle-ci sur un polymère soluble dans l'eau choisi dans le groupe de polysaccharide, polyéthylèneglycol, polyvinylpyrrolidone ou polyacide anydrique.

5. Procédé selon la revendication 4, caractérisé en ce qu'il se produit une liaison sur un polymère activé.

6. Procédé selon la revendication 4 ou 5, caractérisé en ce que l'on utilise un polysaccharide comme polymère.

7. Procédé selon la revendication 4, caractérisé en ce que l'on utilise l'éthylènediamine comme alkylènediamène et en ce que l'on utilise un polysaccharide activé comme polymère.

8. Utilisation d'un dérivé de peroxydase soluble dans l'eau selon l'une quelconque des revendications 1 à 3 pour la détermination de $H_2O_2$.